# EUROPÄISCHE PATENTANMELDUNG

(11) **EP 3 338 812 A1**
(43) Veröffentlichungstag der Anmeldung: **27.06.2018**
(21) Anmeldenummer: 17209127.4
(22) Anmeldetag: 20.12.2017
(51) Int. Cl.: A61L 12/06, A61L 2/10, A61L 12/02, A61L 2/025

(54) **VERFAHREN UND VORRICHTUNG ZUR REINIGUNG EINES MEDIZINPRODUKTS**

(30) Priorität: 20.12.2016 DE 102016225596
(71) Anmelder: Ernst-Moritz-Arndt-Universität Greifswald, 17489 Greifswald (DE); Teleaugendienst GmbH, 17475 Greifswald (DE)
(72) Erfinder: Großjohann, Rico, 17498 Mesekenhagen (DE); Tost, Frank, 17489 Greifswald (DE)
(74) Vertreter: Ramrath, Lukas

(57) **Zusammenfassung**

Die Erfindung betrifft ein Verfahren und eine Vorrichtung (1) zur Reinigung eines Medizinprodukts, die Vorrichtung (1) mindestens einen Aufnahmebereich (3) für das Medizinprodukt aufweist, wobei die Vorrichtung (1) eine erste Strahlungsquelle (11) umfasst, wobei durch die erste Strahlungsquelle (11) Strahlung mit einer Wellenlänge aus einem ersten Wellenlängenbereich von 165 nm bis 230 nm emittierbar ist, wobei zumindest ein Teil der Strahlung der ersten Strahlungsquelle (11) in zumindest einen Teilbereich des Aufnahmebereichs (3) strahlbar ist, wobei die Vorrichtung (1) mindestens eine zweite Strahlungsquelle (13) umfasst, dadurch gekennzeichnet, dass durch die zweite Strahlungsquelle (13) Strahlung mit einer Wellenlänge aus einem zweiten Wellenlängenbereich von 260 nm bis 300 nm emittierbar ist, wobei zumindest ein Teil der Strahlung der zweiten Strahlungsquelle (13) in zumindest einen Teilbereich des Aufnahmebereichs (3) strahlbar ist, wobei die Anordnung zur Steigerung der Effizienz um eine weitere Strahlungsquelle (17) aus einem weiteren UV-Strahlungsbereich von 230 nm bis 260 nm ergänzt werden kann, die in zumindest einen Teilbereich des Aufnahmebereiches (3) emittiert.

## Beschreibung

Die Erfindung betrifft eine Vorrichtung und ein Verfahren zur Reinigung eines Medizinprodukts.

Es ist bekannt, dass Medizinprodukte vor der Anwendung gereinigt werden müssen. So sind auch ophthalmologische Medizinprodukte, insbesondere auch Bedarfsgegenstände mit direktem Augenkontakt, einer regelmäßigen Reinigung zu unterziehen. Zu solchen Medizinprodukten zählen u.a. Augenprothesen, refraktive Kontaktlinsen, medizinisch indizierte Kontaktlinsen, kosmetische Kontaktlinsen und Geräte zur Messung ophthalmologischer Parameter, wie z.B. Kontakttonometer, die vorübergehend am Auge, beispielsweise auf der Kornea verbleiben bzw. diese berühren. Solche Medizinprodukte können insbesondere auch wiederverwendbare Medizinprodukte sein.

Zu den häufigsten Bedarfsgegenständen mit direktem Augenkontakt zählen Instrumente, die Augenärzte und Optiker zur Untersuchung des Auges nutzen, sowie weiche und harte Kontaktlinsen. Weiche Kontaktlinsen sind flexibel und passen sich in ihrer Form der Hornhaut an. Dies äußert sich bei vielen Nutzern in vielen Fällen in einem angenehmeren Tragegefühl. Weiche Kontaktlinsen bestehen aus Kunststoff, wie z.B. HEMA oder Silikon-Hydrogel, lagern Wasser in der Regel sehr gut ein und haben aus diesem Grund eine hohe Porosität. Weiche Kontaktlinsen weisen eine sehr große Oberfläche auf, da sie in der Regel schwammartig ausgebildet sind. Dies macht das Reinigen und Pflegen in Hinblick auf pathogene Mikroorganismen besonders schwierig und aufwendig.

Es ist allgemein bekannt, zum Reinigen, Desinfizieren, Proteinentfernen, Nachbenetzen und Aufbewahren von Medizinprodukten chemische Reinigungsbäder zu verwenden. Je nach Anzahl der Bäder bzw. Wirkstufen werden sogenannte Ein- und Mehrkomponenten-Pflegelösungen unterschieden. Mehrkomponenten-Pflegelösungen, die u.a. auch Peroxide enthalten können, reinigen in der Regel ausreichend gründlich, allerdings kann es hierbei zu Verwechslungen von einzelnen Komponenten kommen, sodass infolge chemisch-toxischer Reaktionen eine gesundheitliche Beeinträchtigung auftreten kann. Aus diesem Grunde bieten viele Hersteller alternativ Einkomponenten-Pflegelösungen an.

Diese beinhalten eine Vielzahl unterschiedlicher Substanzen und Wirkprinzipien, die allerdings möglichst nicht interagieren sollten. Bei diesen Einkomponenten-Pflegelösungen besteht jedoch ein höheres Risiko für verbleibende Verunreinigungen und Verkeimungen. Diese Verkeimungen können u.a. auch auf Resistenzen einzelner Mikroorganismen gegenüber bestimmter (chemischer) Wirksubstanzen, die häufig in geringen Konzentrationen vorhanden sind, zurückgeführt werden.

Ein weiteres Problem der Einkomponenten-Pflegelösung kann in der regelmäßig erfolgenden Zugabe von Konservierungsstoffen bzw. deren zunehmender Konzentration in der Kontaktlinse in Abhängigkeit von der Nutzungsdauer bestehen. Konservierungsstoffe können Allergien auslösen.

Bei Bedarfsgegenständen mit direktem Augenkontakt können chemische Reinigungslösungen außerdem zu Wechselwirkungen führen, die insbesondere die optischen Eigenschaften dieser Gegenstände beeinflussen.

Es ist bekannt, eine Reinigung von Medizinprodukten mit Ultraviolett (UV) Strahlung vorzunehmen. So beschreibt US 2011/0315893 A1 eine Strahlungs-Desinfektions-Basis, die in Zusammenarbeit mit einem Aufnahmebehälter für eine ophthalmologische Linse wirkt. Allerdings offenbart diese Druckschrift nur die Verwendung einer Wellenlänge aus einem Wellenlängenbereich von 250 nm bzw. ungefähr 280 nm.

In anderen Druckschriften fanden in den dafür genutzten UV-Lampen insbesondere die energetisch sehr effiziente Anregung von Quecksilberlinienstrahlung von 253,652 nm (im Weiteren auf 254 nm gerundet) einen vielfachen Einsatz. Zur Steigerung der Strahlungsleistung wurde u.a. der Betriebsdruck dieser Quecksilberlampen variiert. Mit der Erhöhung des Betriebsdruckes dieser Lampen geht gleichsam eine gewisse Verbreiterung der diskreten Quecksilberlinie einher, so dass ein gewisses Spektrum um die Wellenlänge von 254 nm erzeugt wird. Man spricht hierbei von Quecksilber-Mittel- und Hochdruckstrahlern, die infolge der hohen Strahlungsleistung für die UV-Desinfektion von Trink-und Abwässern, sowie die Flächendesinfektion ganzer Räume (bspw. Reinräume) genutzt werden. Bei der Trinkwasserdesinfektion werden in Deutschland derzeit 0,4 mJ/mm² gefordert.

Allerdings decken auch diese intensiven Quecksilber-Mittel- oder Hochdruckstrahler nicht alle Anforderungen der Desinfektion von Medizinprodukten ab. Es ist bekannt, dass pathogene Mikroorganismen UV-Desinfektionsresistenzen aufweisen können. Außerdem sind fast alle pathogenen Mikroorganismen in der Lage auf UV-Bestrahlungsstress mit DNA-Reparaturmechanismen (bspw. Photoreaktivierung oder Dunkelreparatur) zu antworten, so dass eine Toleranz gegenüber physikalischen Desinfektionsmaßnahmen auftreten kann. Beispielhaft seien die Adenoviren genannt, deren zahlreiche Stämme in 6 Untergruppen gegliedert werden und im Allgemeinen als UV-resistent gelten. In der Augenheilkunde sind diese hochinfektiösen Adenoviren häufige für virale Bindehautentzündungen ursächlich, die in der Praxis nicht selten zur endemischen Keratokonjunktivits führen können.

Im Verlauf der Evolution haben Mikroorganismen gelernt sich an veränderte Umweltbedingungen anzupassen. So sind unterschiedliche Reparaturmechanismen der Mikroorganismen auf subzellulärer Ebene bekannt, die einer geschädigten Zelle das Überleben bzw. die weitere Fortpflanzung ermöglichen. Diese Reparaturmechanismen werden prinzipiell in zwei Gruppen unterteilt: die Dunkelreparatur (mit den Unterarten: Nucleotid-Excisions-Reparatur; Rekombinations-Reparatur oder SOS-Reparatur) sowie die Photoreaktivierung. Auch der Prozess der Photoreaktivierung, der über den Prozess der Photolyse abläuft, ist in wissenschaftlichen Veröffentlichungen beschrieben worden. Für die vorliegende Offenbarung wichtig sind eine speziesabhängige Aktivierungsenergie und die Abhängigkeit von der Wellenlänge. Die Photolyse beginnt mit einer minimalen Wellenlänge von etwa 310 nm, so dass man davon ausgehen kann, dass einige bekannte Bestrahlungsquellen wie beispielsweise Quecksilber-Mitteldrucklampen neben der erwünschten Schädigung der Zellen, gleichsam die notwendigen Aktivierungsenergien für den Prozess der Photokatalyse liefern.

Unter Berücksichtigung dieser Betrachtungen sollte eine effektive Desinfektion bzw. Reinigung nicht nur mit Hilfe einer einzigen UV-Strahlung mit einer definierten Wellenlänge von bspw. 254 nm nur einen bestimmten Schädigungstyp bewirken, sondern gleichzeitig breitbandiger wirken und mehrere Schädigungsmechanismen an unterschiedlichen Genloci auslösen.

Die hier vorliegende neuartige Betrachtung der UV-Desinfektion versucht der Gesamtheit dieser Prozesse Rechnung zu tragen, die sich in einem differenzierten und nicht singulären Zelldeaktivierungsspektrum mit lokalen Maxima bei etwa 220 nm und etwa 266 nm darstellen. Aus der wissenschaftlichen Literatur sind einige systematische Untersuchungen der Zelldeaktivierungsspektren oberhalb von 200 nm bekannt. Hierzu ist anzumerken, dass die jeweilig verwendete Messapparaturen zur spektroskopischen Untersuchung einen Wellenlängenbereich und somit eine untere/ minimale Wellenlänge vorgeben. Systematische Untersuchungen der spektralen Zelldeaktivierung unterhalb von 200 nm sind im vorliegenden Kontext bislang rar.

Daraus stellt sich das technische Problem, eine Vorrichtung und ein Verfahren zur Reinigung eines Medizinproduktes, insbesondere eines Bedarfsgegenstandes mit direktem Augenkontakt, zu schaffen, welche eine energetisch effektive, auf vielfältige pathogene Mikroorganismen spezifisch anwendbare, verbesserte Reinigung des Medizinproduktes ermöglichen.

Die Lösung des technischen Problems ergibt sich durch die Gegenstände mit den Merkmalen der Ansprüche 1,13 und 15. Weitere vorteilhafte Ausgestaltungen der Erfindung ergeben sich aus den Unteransprüchen.

Vorgeschlagen wird eine Vorrichtung zur Reinigung eines Medizinprodukts. Ein Medizinprodukt kann insbesondere einen Gegenstand bezeichnen, der zu medizinisch therapeutischen oder diagnostischen Zwecken für Menschen verwendet wird. Das Medizinprodukt kann ein ophthalmologisches Medizinprodukt sein, insbesondere ein (Bedarfs-)Gegenstand zur Verwendung mit direktem Augenkontakt sein. Beispielhafte Medizinprodukte wurden eingangs erläutert.

Die Vorrichtung weist mindestens einen Aufnahmebereich für das Medizinprodukt auf. Im Sinne dieser Erfindung kann "aufweisen" auch bedeuten, dass die Vorrichtung den Aufnahmebereich ausbildet. In dem Aufnahmebereich kann das Medizinprodukt zur Aufbewahrung und insbesondere zur Reinigung angeordnet werden.

Die Vorrichtung ist darauf ausgerichtet, bestimmte pathogene Mikroorganismen gleichzeitig und vorhersehbar auf mehrfache Weise und unterschiedlichen Lokalisationen zu schädigen (Stress), um die Wahrscheinlichkeit erfolgreicher Reparaturmechanismen des pathogenen Mikroorganismus zu minimieren bzw. zu verhindern.

Eine zur Reinigung verwendete UVC-Strahlung kann in drei Wellenlängebereiche unterteilt werden:
1. UVC-I: >165 nm bis ≤ 230 nm
2. UVC-III: > 260 nm bis ≤ 300 nm
3. UVC-II: > 230 nm bis ≤ 260 nm.

Diese speziesspezifischen und prozessspezifischen UVC-Teilbereiche können einzelnen Strahlungsquellen zugeordnet werden. In vorteilhafter Weise steigert die Kombination und Variation der Wellenlängen der UVC-Teilbereiche, die Wirksamkeit der Desinfektion bestimmter pathogener Mikroorganismen.

Die Vorrichtung umfasst mindestens eine erste Strahlungsquelle, wobei durch die erste Strahlungsquelle Strahlung mit einer Wellenlänge aus dem ersten Wellenlängenbereich (UVC-I) von 165 nm (ausschließlich) bis 230 nm (einschließlich) emittierbar ist. Bevorzugt ist eine Wellenlänge aus einem ersten Wellenlängenbereich von 180 nm (einschließlich) bis 225 nm (einschließlich) emittierbar. Besonders bevorzugt wird jedoch keine Wellenlänge, insbesondere auch nicht die Wellenlänge von 253,652 nm.

Grundsätzlich sollte der Öffnungswinkel des jeweiligen UV-Strahlers der zu bestrahlenden Fläche des zu reinigenden Objekts/Medizinprodukts angepasst sein. Diese Anordnung kann in vorteilhafter Weise den Abstand zwischen UV-Strahler und dem zu reinigenden Objekt/Medizinprodukt reduzieren oder minimieren, wodurch die Absorption (infolge des Abstandes), die den Reinigungseffekt mindert, minimiert wird. Auf diese Weise kann in einigen Fällen auf eine Strahlführung verzichtet werden, so dass sich die effektive Bestrahlung optimiert.

Zumindest ein Teil der Strahlung der ersten Strahlungsquelle ist in den Aufnahmebereich oder in zumindest einen Teilbereich des Aufnahmebereichs strahlbar. Insbesondere kann die erste Strahlungsquelle derart ausgebildet und/oder angeordnet sein, dass diese in einen Teil der Strahlung in den Aufnahmebereich oder Teilbereich strahlt. Alternativ oder kumulativ können, wie nachfolgend noch näher erläutert, auch Mittel zur Strahlführung und/oder -formung vorgesehen sein, die Strahlung der ersten Strahlungsquelle in den Teilbereich oder zumindest einen Teilbereich des Aufnahmebereichs lenken.

Weiter umfasst die Vorrichtung mindestens eine zweite Strahlungsquelle.

Erfindungsgemäß ist durch die zweite UV-Strahlungsquelle Strahlung mit einer Wellenlänge aus dem zweiten Wellenlängenbereich (UVC-III) von 260 nm (ausschließlich) bis 300 nm (einschließlich) emittierbar, wobei zumindest ein Teil der Strahlung der zweiten UV-Strahlungsquelle in den Aufnahmebereich oder zumindest einen Teilbereich des Aufnahmebereichs strahlbar ist. Bevorzugt ist eine Wellenlänge aus dem Wellenlängenbereich von 262 nm (ausschließlich) bis 285 nm (einschließlich) emittierbar. Insbesondere kann auch die zweite UV-Strahlungsquelle derart angeordnet und/oder ausgebildet sein, dass Strahlung der zweiten UV-Strahlungsquelle in den Aufnahmebereich oder in zumindest einen Teilbereich des Aufnahmebereichs strahlbar ist. Alternativ oder kumulativ können auch die vorhergehend erläuterten Mittel zur Strahlführung und/oder -formung oder weitere Mittel zur Strahlführung und/oder -formung vorgesehen sein, die Strahlung der zweiten Strahlungsquelle in den Aufnahmebereich oder den zumindest einen Teilbereich des Aufnahmebereichs lenken.

Zur Effizienzsteigerung kann die Vorrichtung mindestens eine weitere, also eine dritte, Strahlungsquelle umfassen. Hierbei kann durch die (mindestens eine) weitere Strahlungsquelle Strahlung mit einer Wellenlänge aus dem dritten Wellenlängenbereich (UVC-II) von 230 nm (ausschließlich) bis 260 nm (einschließlich) emittierbar sein, wobei zumindest ein Teil der Strahlung der weiteren Strahlungsquelle in den Aufnahmebereich oder in zumindest einen Teilbereich des Aufnahmebereichs strahlbar ist. Bevorzugt ist eine Wellenlänge aus dem Wellenlängenbereich von 243 nm (ausschließlich) bis 256 nm (einschließlich) durch die mindestens eine weitere Strahlungsquelle. Auch kann die mindestens eine weitere UV-Strahlungsquelle derart angeordnet und/oder ausgebildet sein, dass Strahlung der weiteren UV-Strahlungsquelle in den Aufnahmebereich oder zumindest einen Teilbereich des Aufnahmebereichs strahlbar ist. Alternativ oder kumulativ können auch die vorhergehend erläuterten Mittel zur Strahlführung und/oder -formung oder weitere Mittel zur Strahlführung und/oder -formung vorgesehen sein, die Strahlung der weiteren Strahlungsquelle in den Aufnahmebereich oder zumindest einen Teilbereich des Aufnahmebereichs lenken.

Vorzugsweise entsprechen sich die bestrahlbaren Bereiche oder Teilbereiche der mindestens zwei Strahlungsquellen oder sie überlagern sich zumindest teilweise. Allerdings ist es auch vorstellbar, dass die bestrahlbaren Bereiche oder Teilbereiche vollständig voneinander verschieden sind.

Die erste und/oder die zweite und/oder die mindestens eine weitere Strahlungsquelle können auch als Flächenstrahler ausgebildet sein. Vorzugsweise ist die zweite Strahlungsquelle als Flächenstrahler ausgebildet. In diesem Fall können die erste und die mindestens eine weitere Strahlungsquelle nicht als Flächenstrahler ausgebildet sein. Es ist jedoch möglich, dass in diesem Fall die erste und/oder die mindestens eine weitere Strahlungsquelle als Flächenstrahler ausgebildet sind.

In diesem Fall kann die Strahlung flächig angepasst in den Aufnahmebereich gestrahlt werden. Bei einem Flächenstrahler kann die erzeugte Strahlung von einer Fläche mit vorbestimmter Größer abgegeben werden, insbesondere in den Aufnahmebereich oder Teilbereich. Die Größe der Fläche kann beispielsweise 4 mm² bis 40.000 mm² betragen, insbesondere für eine Bestrahlung von Bedarfsgegenständen mit direktem Augenkontakt.

Ein Flächenstrahler kann hierbei auch durch mindestens einen Punktstrahler und mindestens ein Mittel zur Strahlführung und/oder - formung, beispielsweise eine Linse oder ein Strahlaufweiter oder -streuer, gebildet werden, wobei vom Punktstrahler erzeugte Strahlung und von dem mindestens einen Mittel zur Strahlführung und/oder-formung geführte/geformte Strahlung dann von der Fläche mit vorbestimmter Größe in den Aufnahmebereich oder Teilbereich abgegeben wird.

Ein Flächenstrahler kann hierbei auch durch eine Gesamtheit von mindestens zwei Punktstrahlern, die Strahlung der gleichen Wellenlänge emittieren, gebildet werden. Hierbei kann die Fläche, von der Strahlung abgegeben wird, die Abstrahlabschnitte der Punktstrahler umfasst. Ein Abstrahlabschnitt kann hierbei weniger als ein vorbestimmter Abstand, beispielsweise weniger als 5 mm, vorzugsweise weniger als 2 mm, von dem Abstrahlabschnitt des am nächsten benachbarten Punktstrahlers angeordnet sein. Der Abstand kann hierbei in der Ebene der Fläche erfasst werden. Auch in diesem Fall kann der Flächenstrahler mindestens ein Mittel zur Strahlführung und/oder - formung, umfassen, wobei von einem, mehreren oder allen Punktstrahlern erzeugte Strahlung und von dem mindestens einen Mittel zur Strahlführung und/oder - formung geführte/geformte Strahlung dann von der Fläche mit vorbestimmter Größe in den Aufnahmebereich oder Teilbereich abgegeben wird.

Durch die spezifische Wahl der UV-Teilbereiche und die Kombination der UV-Strahlungsquellen kann angenommen werden, dass die Kombination der Strahlung mit der Wellenlänge aus dem ersten Wellenlängenbereich , die auch als erste Wellenlänge bezeichnet werden kann, mit der Strahlung mit der Wellenlänge aus dem zweiten Wellenlängenbereich, die auch als zweite Wellenlänge bezeichnet werden kann, und gegebenenfalls, aber nicht zwingend, mit der Strahlung aus dem dritten, weiteren Wellenlängenbereich, verschiedene effektivere Wirkungen auf relevante Bakterien, wie z.B. Staphylococcus aureus, Serratia marcescens und Pseudomonas aeruginosa, auf relevante Viren, bspw. Adenoviren und Pilze ausüben. Insbesondere können durch die verschiedenen Wellenlängen voneinander verschiedene Basenpaare einer Zelle eines Bakteriums, des Virus (oder ggf. dessen Hülle) oder eines Pilzes effektiv zerstört werden.

Durch die neuartige Kombination von Strahlung aus mindestens zwei UV-Wellenlängenbereichen, vorzugsweise aus den drei dargestellten UV-Teilbereichen, werden Zellen bzw. die DNA auf mehrfache Weise unterschiedlich geschädigt und somit effizienter gestresst. Um zu überleben müsste die Zelle gleichzeitig mehrere unterschiedliche Reparaturmechanismen starten und erfolgreich beenden. Die Überlebenswahrscheinlichkeit der Mikroorganismen sinkt. Auf diese Weise wird in vorteilhafter Weise in einem Energiebereich deutlich unterhalb der bekannten Quecksilber-Mittel- und Hochdruckstrahler die Reinigungswirkung entscheidend verbessert und insbesondere die Reinigungseffektivität erhöht.

So konnte in Versuchen gezeigt werden, dass die Anzahl der vorhergehend genannten relevanten Bakterien um mehr als fünf log10-Stufen reduziert werden kann. Auch ophthalmologisch relevante Pilze, wie z.B. Candida albicans und Fusarium solani, können in einer für Bedarfsgegenstände mit direktem Augenkontakt akzeptablen Bestrahlungszeit um mehr als zwei log10-Stufen reduziert werden.

Weiter konnte gezeigt werden, dass eine deutlich höhere Reduktion bei der Verwendung der ersten und der zweiten Wellenlänge als bei der Verwendung von Strahlung mit nur einer Wellenlänge aus dem ersten Wellenlängenbereich möglich ist. Die Verwendung der Strahlung mit einer zweiten Wellenlänge aus dem zweiten Wellenlängenbereich ist für ein Medizinprodukt, insbesondere im Bereich der Ophthalmologie, überraschend, weil bekannte Verfahren die UV Desinfektion mit einer einzigen, definierten Wellenlänge (meist 254 nm) beschreiben.

Durch die Verwendung der Strahlung mit verschiedenen Wellenlängen ergibt sich weiterhin in vorteilhafter Weise eine materialschonendere Reinigung, da das Material des, insbesondere ophthalmologischen, Medizinprodukts durch die Verwendung von mindestens zwei Wellenlängen weniger beeinflusst wird als bei der Verwendung nur einer Wellenlänge höherer Intensität, wenn der gleiche Reinigungseffekt erzielt werden soll.

Vorzugsweise umfasst die Vorrichtung eine erste Menge von mindestens zwei Strahlungsquellen, wobei jede Strahlungsquelle der ersten Menge Strahlung mit einer Wellenlänge aus dem ersten Wellenlängenbereich emittieren kann. Diese Wellenlängen können voneinander verschieden sein. Es ist möglich, dass kein oder mindestens eine Strahlungsquelle der ersten Menge ein Flächenstrahler ist.

Weiter vorzugsweise umfasst die Vorrichtung eine zweite Menge von mindestens zwei Strahlungsquellen, wobei jede Strahlungsquelle der zweiten Menge Strahlung mit einer Wellenlänge aus dem zweiten Wellenlängenbereich emittieren kann. Diese Wellenlängen können voneinander verschieden sein. Es ist möglich, dass kein oder mindestens eine Strahlungsquelle der zweiten Menge ein Flächenstrahler ist. Vorzugsweise ist mindestens eine Strahlungsquelle der zweiten Menge ein Flächenstrahler.

Ebenfalls kann die Vorrichtung eine dritte Menge von mindestens zwei Strahlungsquellen umfassen, wobei jede Strahlungsquelle der dritten Menge Strahlung mit einer Wellenlänge aus dem dritten Wellenlängenbereich emittieren kann. Diese Wellenlängen können voneinander verschieden sein. Es ist möglich, dass kein oder mindestens eine Strahlungsquelle der dritten Menge ein Flächenstrahler ist.

Die erste Menge, die zweite Menge und/oder die dritte Menge können beispielsweise 4 oder 5 Strahlungsquellen umfassen. Es ist möglich, dass die Mengen eine unterschiedliche Anzahl von Strahlungsquellen umfassen.

Die Intensität der von einer Strahlungsquelle emittierbaren Strahlung kann einstellbar, also variabel, sein.

Weiter können die Strahlungsquellen der Vorrichtung, insbesondere auch der ersten Menge, der zweiten Menge und/oder der dritten Menge, aktivierbar und deaktivierbar sein. So ist es möglich, dass in einem Verfahren zur Reinigung eines Medizinprodukts oder eines Gehäuses der Vorrichtung zur Reinigung eines Medizinprodukts nur eine, mehrere, aber nicht alle oder aber alle Strahlungsquellen der Vorrichtung oder der ersten Menge von Strahlungsquellen, der zweiten Menge von Strahlungsquellen und/oder der dritten Menge von Strahlungsquellen aktiviert sind, insbesondere in einem bestimmten Betriebsmodus der Vorrichtung. Weiter können die Intensitäten der Strahlung der aktivierten Strahlungsquellen auf vorbestimmte Intensitäten eingestellt werden.

Insbesondere ist es möglich, nur Strahlungsquellen zu aktivieren, die Strahlung mit einer vorbestimmten Wellenlänge, insbesondere aus den erläuterten Wellenlängenbereichen, emittieren. Mit anderen Worten ist die Vorrichtung in mindestens zwei voneinander verschiedenen Betriebsmodi betreibbar, wobei sich verschiedene Betriebsmodi in dem Aktivierungszustand mindestens einer Strahlungsquelle, insbesondere der ersten und/oder der zweiten und/oder der dritten Menge von Strahlungsquellen, und/oder in der Intensität der von einer ausgewählten Strahlungsquelle im jeweiligen Betriebsmodus emittierten Strahlung unterscheiden. Mit anderen Worten kann in voneinander verschiedenen Betriebsmodi eine Bestrahlung mit verschiedenen Wellenlängen und/oder verschiedenen Intensitäten, insbesondere wellenlängenspezifischen Intensitäten, erfolgen.

Somit kann eine Bestrahlung insbesondere spezienspezifisch erfolgen. Insbesondere kann ein Betriebsmodi gezielt spezienspezifisch sein. Insbesondere kann die Vorrichtung also einem Betriebsmodus betrieben werden, der eine möglichst effektive Schädigung bzw. Zerstörung einer bestimmten Spezies bzw. Gruppe von Spezies ermöglicht. Durch eine derartige spezienspezifische Bestrahlung ist es im Vergleich mit einer spezienunspezifischen Bestrahlung möglich, die Intensitäten der emittierten Strahlung, insbesondere die Summe aller Intensitäten, zu reduzieren. Dies wiederum ermöglicht in vorteilhafter Weise die Schonung des bestrahlten Materials des Medizinprodukts als auch der Vorrichtung.

Weiter ergibt sich durch die vorgeschlagene Vorrichtung in vorteilhafter Weise eine kontaktfreie Reinigung des Medizinprodukts.

In einer weiteren Ausführungsform umfasst die Vorrichtung mindestens eine weitere Strahlungsquelle, wobei durch die mindestens eine weitere Strahlungsquelle Strahlung mit einer Wellenlänge aus einem dritten Wellenlängenbereich von 230 nm (ausschließlich) bis 260 nm (einschließlich) emittierbar ist, wobei zumindest ein Teil der Strahlung der mindestens einen weiteren Strahlungsquelle in den Aufnahmebereich oder zumindest einen Teilbereich des Aufnahmebereichs strahlbar ist. Dies und entsprechende Vorteile wurden vorhergehend erläutert.

In einer weiteren Ausführungsform ist die erste Strahlungsquelle als UV-LED (Ultraviolett-Leuchtdiode) ausgebildet. Alternativ ist die erste Strahlungsquelle als UV-OLED (Ultraviolett- organische Leuchtdiode) ausgebildet. Weiter alternativ ist die erste Strahlungsquelle als UV-Laserquelle ausgebildet.

Alternativ oder kumulativ ist die zweite Strahlungsquelle als UV-OLED ausgebildet. Alternativ ist die zweite Strahlungsquelle als UV-LED ausgebildet. Weiter alternativ ist die zweite Strahlungsquelle als UV-Laserquelle ausgebildet.

Wird mindestens eine weitere Strahlungsquelle verwendet, so kann diese als UV-OLED oder als UV-LED oder als UV-Laserquelle ausgebildet sein.

Bevorzugt sind die erste sowie auch die weitere(n) Strahlungsquelle(n) jeweils als UV-LED ausgebildet. Allerdings ist es auch vorstellbar, dass sowohl die erste Strahlungsquelle als auch die weitere(n) Strahlungsquelle(n) als UV-OLED ausgebildet sind. Eine OLED kann insbesondere als Flächenstrahler bzw. ein Flächenstrahler kann als OLED ausgebildet sein. Hierbei wird die von der UV-OLED erzeugte Strahlung von einer Fläche mit vorbestimmter Größer abgegeben. Die Größe einer UV-OLED kann für Bedarfsgegenstände mit direktem Augenkontakt beispielsweise 4 bis 40.000 mm² betragen. Eine UV-LED oder eine UV-Laserquelle kann insbesondere als Punktstrahler bzw. ein Punktstrahler kann als UV-LED oder UV-Laserquelle ausgebildet sein.

Durch die Verwendung einer UV-LED ergibt sich in vorteilhafter Weise ein schmalbandige spezifische Strahlung. Diese Strahlung kann gegebenenfalls für einen bestimmten pathogenen Mikroorganismus auch mit einer Wellenlänge unterhalb einer Wellenlänge von 230 nm erzeugt werden.

Durch die Verwendung einer UV-OLED ergibt sich in vorteilhafter Weise eine effiziente Flächenstrahlung. Die Verwendung von UV-OLED ist insbesondere daher überraschend, da aktuell die Verwendung von UV-OLED zur Reinigung eines ophthalmologischen Medizinproduktes mit einer Strahlung aus dem ersten und/oder dem zweiten und gegebenenfalls dem dritten Wellenlängenbereichen nicht bekannt ist.

Durch die Verwendung einer UV-Laserquelle ergibt sich in vorteilhafter Weise eine monochromatische spezifische Strahlung. Diese Strahlung kann gegebenenfalls für einen bestimmten pathogenen Mikroorganismus auch mit einer Wellenlänge unterhalb einer Wellenlänge von 230 nm erzeugt werden. Weiter lässt sich die Strahlung durch Strahlführung relativ leicht auf die geometrischen Gegebenheiten des zu reinigenden Objektes anpassen (vereinfachte Strahlführung).

Eine UV-LED und eine UV-Laserquelle können insbesondere als Punktstrahler oder nahezu als Punktstrahler ausgebildet sein.

In einer weiteren Ausführungsform ist eine Summe der von aktivierten oder aktivierbaren Strahlungsquellen emittierbaren Strahlung größer als oder gleich 2,0 µJ/mm². Alternativ oder kumulativ kann durch mindestens eine Strahlungsquelle Strahlung mit einer Strahlungsenergie von mindestens 0,1 µJ/mm² emittierbar sein. Die Strahlungsenergie kann hierbei auch kumulativ, also durch mehr als eine Strahlungsquelle, erzeugt werden. In diesem Fall kann also die Strahlungsenergie die summierte Strahlungsenergie aller Strahlungsquellen bezeichnen. Die maximale Strahlungsenergie kann auf das zu reinigende Medizinprodukt angepasst werden. Die Unterschiede hinsichtlich der maximalen Strahlungsenergie beispielsweise zwischen einer Kontaktlinse und einem zu reinigendem Okular unterscheidet sich erheblich und können einige Größenordnungen betragen. Hierdurch wird in vorteilhafter Weise gewährleistet, dass eine besonders zuverlässige Reduktion relevanter Bakterien und Pilze des kontaminierten und zu reinigenden Objektes erfolgt.

Die Einstrahlung in den Aufnahmebereich oder jeweiligen Teilbereich kann hierbei zumindest zeitweise gleichzeitig oder sequenziell erfolgen. So ist es vorstellbar, dass zu einem bestimmten Betriebszeitpunkt nur eine von mehreren Strahlungsquellen aktiviert ist.

In einer weiteren Ausführungsform umfasst die Vorrichtung zusätzlich eine Ultraschallquelle, wobei zumindest ein Teil des Ultraschallsignals in den Aufnahmebereich oder zumindest einen Teilbereich des Aufnahmebereichs strahlbar ist. Insbesondere kann die mindestens eine Ultraschallquelle derart angeordnet und/oder ausgebildet sein, dass Ultraschall in den Aufnahmebereich gestrahlt werden kann. Selbstverständlich können auch Mittel zur Ultraschallführung und/oder -formung vorhanden sein, mittels derer Ultraschall in den Aufnahmebereich gelenkt werden kann. Der vom Ultraschallsignal bestrahlbare Teilbereich kann hierbei dem oder den von den Strahlungsquellen bestrahlbaren Teilbereich(en) entsprechen oder sich zumindest teilweise mit diesem/diesen überlagern.

Es ist bekannt, dass durch den Ultraschall in vorteilhafter Weise insbesondere grobe Schmutzpartikel und grobe Proteinstrukturen von dem Medizinprodukt entfernt werden können. Somit erhöht sich in Kombination mit der zuvor erläuterten UV-Strahlungsquellen in vorteilhafter Weise der Reinigungsgrad.

In einer bevorzugten Ausführungsform umfasst die Vorrichtung mindestens ein Mittel zur Strahlführung und/oder -formung.

Durch ein Mittel zur Strahlführung kann eine Strahlrichtung geändert werden. Das Mittel zur Strahlführung kann insbesondere als Reflektor ausgebildet sein, der einfallende Strahlung unter einem gewünschten Reflexionswinkel zumindest teilweise oder vollständig reflektiert.

Durch ein Mittel zur Strahlformung kann einfallende Strahlung gebündelt und/oder gestreut werden. Durch ein Mittel zur Strahlführung kann insbesondere eine Fokussierung oder eine Aufweitung der Strahlung erfolgen. Somit kann eine Bestrahlung des Medizinprodukts in Form einer Spotbestrahlung oder in Form einer Flächenbestrahlung erfolgen.

Weiter kann eine die Veränderung mindestens einer Strahleigenschaft durch das Mittel zur Strahlführung und/oder -formung einstellbar sein. Diese Veränderung kann beispielsweise manuell und/oder aktorgestützt erfolgen. So kann z.B. eine Position und/oder Ausrichtung des Mittels zur Strahlführung und/oder -formung veränderbar sein. Beispielsweise kann das Mittel zur Strahlführung als ein um eine oder mehrere Achse(n) verdrehbarer Reflektor ausgebildet sein. Auch kann die Art der Strahlformung durch das Mittel zur Strahlformung veränderbar sein.

Hierdurch ergeben sich in vorteilhafter Weise eine Vergrößerung des bestrahlbaren Bereichs oder Teilbereichs und/oder eine einstellbare räumlich gezielte oder räumlich verteilte Bestrahlung.

In einer weiteren Ausführungsform ist der Aufnahmebereich zumindest einseitig durch ein Wandelement begrenzt. Das Wandelement bezeichnet ein Element, welches den Aufnahmebereich zumindest teilweise ein- oder umfasst. Die mindestens eine Strahlungsquelle ist im oder am Wandelement angeordnet. Das Wandelement kann hierbei zumindest teilweise durchlässig für die erzeugte Strahlung sein. Das Wandelement kann insbesondere aus UV-transparentem Quarzglas ausgebildet sein.

Vorstellbar ist, dass die Strahlungsquelle vollständig in dem Wandelement eingebettet ist. Auch kann das Wandelement eine Öffnung aufweisen, durch die Strahlung von der Strahlungsquelle in den Aufnahmebereich strahlen kann. Auch kann die Strahlungsquelle an einer Außenseite oder Außenfläche des Wandelements befestigt sein.

Wie nachfolgend noch näher beschrieben kann das Wandelement insbesondere ein Element eines Gehäuses sein. Beispielsweise kann das Wandelement ein Element des Boden- oder Deckelteils sein. Auch kann das Wandelement eine Seitenwand des Aufnahmebereichs ausbilden.

Hierdurch ergibt sich in vorteilhafter Weise eine räumlich nahe Anordnung der Strahlungsquelle am Aufnahmebereich. Da hierdurch eine Intensität der emittierten Strahlung nur minimal reduziert wird, erhöht sich in vorteilhafter Weise die Reinigungswirkung. Beispielhaft kann der Abstand des UV-Strahlers mit angepasstem Öffnungswinkel zum Medizinprodukt kleiner als oder gleich 1 mm betragen.

In einer weiteren Ausführungsform ist eine Bestrahlungsdauer und/oder Bestrahlungsleistung einstellbar. Die Vorrichtung kann hierfür beispielsweise mindestens eine geeignete Eingabeeinrichtung aufweisen. Die Eingabeeinrichtung kann z.B. manuell betätigt werden, um die Bestrahlungsdauer und/oder die Bestrahlungsleistung einzustellen. Hierdurch ergibt sich in vorteilhafter Weise, dass die gewünschte Reinigungswirkung für verschiedene Medizinprodukte angepasst werden kann. Weiter kann in vorteilhafter Weise eine gewünschte Reinigungsdauer eingehalten werden. Kann alternativ, vorzugsweise aber kumulativ, auch die Bestrahlungsleistung eingestellt werden, so kann in vorteilhafter Weise eine Intensität der Reinigung eingestellt werden, z.B. in Abhängigkeit der jeweiligen Spezies. Insbesondere ergibt sich somit auch die Möglichkeit, eine intensive Schnellreinigung oder eine für Medizinprodukte mildere Reinigung über mehrere Stunden einzustellen und gegebenenfalls zu wiederholen.

In einer weiteren Ausführungsform umfasst die Vorrichtung einen Aufnahmebehälter, wobei der Aufnahmebehälter den Aufnahmebereich aufweist. Die erste und die mindestens zweite Strahlungsquelle sind im oder am Aufnahmebehälter angeordnet. Selbstverständlich kann auch eine weitere, dritte Strahlungsquelle im oder am Aufnahmebehälter angeordnet sein. Der Aufnahmebehälter kann von dem vorhergehend erläuterten Gehäuse ausgebildet werden. Beispielsweise kann die Vorrichtung als Box, insbesondere als tragbare Box, ausgebildet sein.

Der Aufnahmebehälter kann einen Deckelteil und einen Bodenteil umfassen. Hierbei kann z.B. nur der Bodenteil den Aufnahmebereich aufweisen. Allerdings können auch sowohl Deckelteil als auch Bodenteil verschiedene Teilbereiche des Aufnahmebereichs aufweisen oder ausbilden.

Weiter kann die erste Strahlungsquelle im oder am Deckelteil und die zweite Strahlungsquelle im oder am Bodenteil angeordnet sein.

Die Vorrichtung kann weiter Mittel zur Befestigung an einer geeigneten Tragestruktur aufweisen. Auch kann die Vorrichtung Mittel zur Befestigung an einem zu reinigenden Gerät, beispielsweise des vorhergehend erläuterten Geräts zur Messung ophthalmologischer Parameter, aufweisen.

Die Vorrichtung kann als portables Gerät ausgebildet sein.

Die Vorrichtung kann mindestens eine Steuereinrichtung zur Steuerung eines Betriebs der Strahlungsquellen, der Ultraschallquelle und/oder der Mittel zur Strahlführung und/oder - formung aufweisen. Weiter kann die Vorrichtung mindestens eine Energiespeichereinrichtung aufweisen, die Energie zum Betrieb der vorhergehend erläuterten Elemente bereitstellt. Die Energiespeichereinrichtung kann insbesondere eine wieder aufladbare Energiespeichereinrichtung sein.

Weiter vorgeschlagen wird ein Verfahren zur Reinigung eines Medizinprodukts. Das Verfahren ist hierbei mit einer Vorrichtung gemäß einer der vorhergehend erläuterten Ausführungsformen durchführbar. Das Medizinprodukt wird in einem Aufnahmebereich angeordnet. Weiter wird Strahlung mit einer Wellenlänge aus einem ersten Wellenlängenbereich von 165 nm (einschließlich) bis 230 nm (einschließlich) in den Aufnahmebereich oder zumindest einen Teilbereich des Aufnahmebereichs eingestrahlt.

Erfindungsgemäß wird Strahlung mit einer Wellenlänge aus einem zweiten Wellenlängenbereich von 260 nm (ausschließlich) bis 300 nm (einschließlich) in den Aufnahmebereich oder zumindest einen Teilbereich des Aufnahmebereichs eingestrahlt.

Die Einstrahlung in den jeweiligen Teilbereich kann hierbei zumindest zeitweise gleichzeitig oder sequenziell erfolgen. So ist vorstellbar, dass zu einem bestimmten Betriebszeitpunkt nur eine der beiden Strahlungsquellen aktiviert ist.

Weiter kann Strahlung mit einer Wellenlänge aus einem dritten Wellenlängenbereich von 230 nm (ausschließlich) bis 260 nm (einschließlich) in den Aufnahmebereich oder zumindest einen Teilbereich des Aufnahmebereichs eingestrahlt werden.

Insbesondere können auch verschiedene Betriebsmodi, insbesondere spezienspezifische Betriebsmodi, der Vorrichtung eingestellt werden.

Umfasst die Vorrichtung eine erste Menge von mindestens zwei Strahlungsquellen, wobei durch jede Strahlungsquelle der ersten Menge Strahlung mit einer Wellenlänge aus dem ersten Wellenlängenbereich emittierbar ist, und/oder eine zweite Menge von mindestens zwei Strahlungsquellen umfasst, wobei durch jede Strahlungsquelle der zweiten Menge Strahlung mit einer Wellenlänge aus dem zweiten Wellenlängenbereich emittierbar ist, so kann im Verfahren zur Reinigung genau eine, mehrere aber nicht alle oder aber alle Strahlungsquelle(n) der ersten Menge und genau eine, mehrere aber nicht alle oder aber alle Strahlungsquelle(n) der zweiten Menge aktiviert werden. Weiter kann genau eine, mehrere aber nicht alle oder aber alle Strahlungsquelle(n) der dritten Menge aktiviert werden. Strahlungsquellen einer Menge können hierbei Strahlung mit voneinander verschiedenen Wellenlängen emittieren. Weiter können Intensitäten der von den aktivierten Strahlungsquellen emittierten Strahlung eingestellt werden.

Die Strahlung mit der ersten Wellenlänge und/oder die Strahlung mit der weiteren Wellenlänge kann hierbei mit einer Strahlungsleistung von mindestens 0,1 µJ/mm² erzeugt werden.

Weiter ist es vorstellbar, dass die Strahlung mit der ersten Wellenlänge und/oder die Strahlung mit der weiteren Wellenlänge durch mindestens ein Mittel zur Strahlführung und/oder -formung derart beeinflusst wird, dass der Aufnahmebereich oder gezielt ein oder mehrere Teilbereiche des Aufnahmebereichs bestrahlt wird/werden. Diese gezielte Bestrahlung mehrerer Teilbereiche kann hierbei gleichzeitig oder sequenziell erfolgen. Hierzu können die erläuterten Mittel zur Strahlführung und/oder -formung entsprechend gesteuert werden.

Weiter kann eine Bestrahlungsdauer eingestellt werden. Hierdurch kann in vorteilhafter Weise eine gewünschte Reinigungsdauer eingehalten werden. Alternativ, vorzugsweise aber kumulativ, kann auch die Bestrahlungsleistung eingestellt werden. Somit kann in vorteilhafter Weise eine Intensität der Reinigung eingestellt werden. Insbesondere ergibt sich somit auch die Möglichkeit, eine intensive Schnellreinigung oder eine mildere Reinigung über mehrere Stunden einzustellen.

Weiter kann ein Ultraschallsignal in den Aufnahmebereich gestrahlt werden. Auch dies kann zumindest zeitweise gleichzeitig mit oder aber sequenziell zu der Bestrahlung mit der ersten, zweiten und/oder der weiteren Wellenlänge erfolgen.

In einer weiteren Ausführungsform wird zusätzlich eine Reinigungslösung im Aufnahmebereich angeordnet. Hierbei kann eine Bestrahlung auch dann erfolgen, wenn keine Reinigungslösung im Aufnahmebereich angeordnet ist. Vorzugsweise erfolgt jedoch eine Bestrahlung auch dann, wenn Reinigungslösung im Aufnahmebereich angeordnet ist. In diesem Fall ergibt sich in vorteilhafter Weise, dass auch ein Teil der Reinigungslösung durch die Strahlung gereinigt wird. Eine Reinigung von Reinigungslösung durch die vorgeschlagene Vorrichtung kann hierbei auch dann erfolgen, wenn zwar Reinigungslösung, jedoch kein Medizinprodukt, im Aufnahmebereich angeordnet ist.

Weiter ergibt sich in vorteilhafter Weise, dass durch die Vorrichtung auch die den Aufnahmebereich ausbildenden Elemente der Vorrichtung gereinigt werden können. Beispielsweise können durch die erzeugte Strahlung Wandelemente, die den Aufnahmebereich einfassen, bestrahlt werden, wodurch diese Wandelemente gereinigt werden. Die Reinigung der den Aufnahmebereich ausbildenden Elemente kann hierbei insbesondere auch dann erfolgen, wenn kein Medizinprodukt im Aufnahmebereich angeordnet ist.

Somit wird auch ein Verfahren zur Reinigung einer Reinigungslösung und/oder der vorgeschlagenen Vorrichtung selbst beschrieben. Das Verfahren ist hierbei mit einer Vorrichtung gemäß einer der vorhergehend erläuterten Ausführungsformen durchführbar.

Insbesondere wird also ein Verfahren zur Reinigung eines Gehäuses einer Vorrichtung zur Reinigung eines Medizinprodukts gemäß einer der in dieser Offenbarung offenbarten Ausführungsformen und/oder zur Reinigung einer Reinigungslösung vorgeschlagen, wobei Strahlung mit einer Wellenlänge aus einem ersten Wellenlängenbereich von 165 nm bis 230 nm in den Aufnahmebereich oder zumindest einen Teilbereich eines Aufnahmebereichs des Gehäuses eingestrahlt wird und/oder Strahlung mit einer Wellenlänge aus einem zweiten Wellenlängenbereich von 260 nm bis 300 nm in den Aufnahmebereich oder zumindest einen Teilbereich des Aufnahmebereichs eingestrahlt wird, wenn kein Gegenstand, insbesondere also kein Medizinprodukt, im Aufnahmebereich angeordnet ist. Allerdings kann Reinigungslösung im Aufnahmebereich angeordnet sein.

Zur Reinigung der Reinigungslösung wird diese im Aufnahmebereich angeordnet. Zur Reinigung der Vorrichtung ist dies jedoch nicht zwingend erforderlich. Weiter wird Strahlung mit einer Wellenlänge aus einem ersten Wellenlängenbereich von 165 nm (ausschließlich) bis 230 nm (einschließlich) in den Aufnahmebereich oder zumindest einen Teilbereich des Aufnahmebereichs eingestrahlt. Weiter wird Strahlung mit einer Wellenlänge aus einem zweiten Wellenlängenbereich von 260 nm (ausschließlich) bis 300 nm (einschließlich) in den Aufnahmebereich oder zumindest einen Teilbereich des Aufnahmebereichs eingestrahlt. Die Reinigung der Vorrichtung nebst Reinigungslösung kann durch Strahlung aus einem weiteren, dritten Wellenlängenbereich (UVC-II; 230 nm (ausschließlich) bis 260 nm (einschließlich)) ergänzt werden.

Durch die Möglichkeit der zeitlich parallelen oder sequenziellen Reinigung des Medizinprodukts, der Pflegelösung und/oder der Vorrichtung selbst können somit in vorteilhafter Weise Energie, Zeit und Kosten eingespart werden.

Die Erfindung wird anhand mehrerer Ausführungsbeispiele näher erläutert. Die Figuren zeigen:
- Fig. 1: einen schematischen Querschnitt durch eine erfindungsgemäße Vorrichtung in einer ersten Ausführungsform,
- Fig. 2: einen schematischen Querschnitt durch eine erfindungsgemäße Vorrichtung in einer zweiten Ausführungsform und
- Fig. 3: einen schematischen Querschnitt durch eine erfindungsgemäße Vorrichtung in einer dritten Ausführungsform.

Nachfolgend bezeichnen gleiche Bezugszeichen Elemente mit gleichen oder ähnlichen technischen Merkmalen.

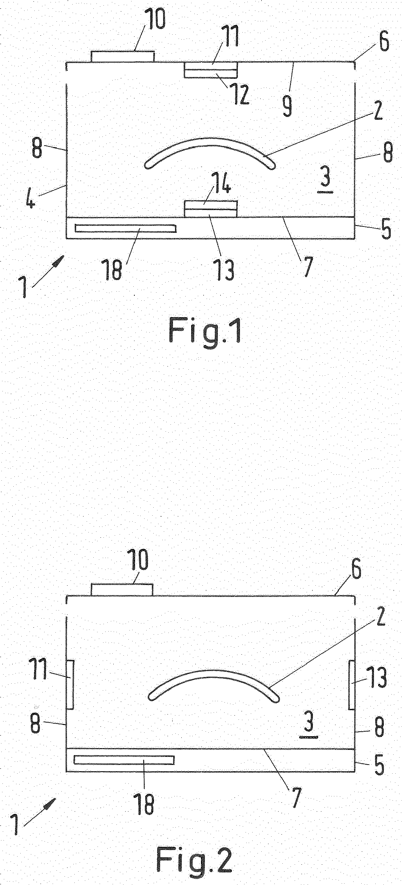

In Fig. 1 ist ein schematischer Querschnitt durch eine erfindungsgemäße Vorrichtung 1 zur Reinigung eines Medizinproduktes, welches exemplarisch für Bedarfsgegenstände mit direktem Augenkontakt als Kontaktlinse 2 ausgebildet ist, dargestellt. Die Vorrichtung 1 umfasst einen Aufnahmebereich 3, der auch als Aufnahmevolumen bezeichnet werden kann. In dem Aufnahmebereich 3 ist die Kontaktlinse 2 angeordnet. Weiter kann in dem Aufnahmebereich 3 eine Reinigungs- oder Pflegelösung angeordnet sein, die die Kontaktlinse 2 umgibt.

Der Aufnahmebereich wird von einem Gehäuse 4 ausgebildet, welches einen Bodenteil 5 und einen Deckelteil 6 umfasst. Der Bodenteil 5 bildet hierbei einen Aufnahmebehälter aus. Hierbei ist dargestellt, dass der Aufnahmebereich 3 ausschließlich oder zu einem großen Teil von dem Bodenteil 5 ausgebildet wird.

In Fig. 1 sind hierbei eine Bodenwand 7, Seitenwände 8 sowie eine Deckenwand 9 dargestellt, die den Aufnahmebereich 3 einfassen oder umgeben. Im unteren Bereich des Bodenteils 5 ist ein Aufnahmebereich für eine Energiespeichereinrichtung 18 angeordnet, wobei die Energiespeichereinrichtung 18 beispielsweise als Akku oder Batterie ausgebildet sein kann. Selbstverständlich kann die Energiespeichereinrichtung 18 auch außerhalb des Gehäuses 4 angeordnet sein.

Weiter dargestellt ist eine Steuer- und Bedieneinrichtung 10, die an einer Außenseite des Deckelteils 6 angeordnet ist. Selbstverständlich kann die Steuer- und Bedieneinrichtung 10 jedoch auch an anderer Stelle des Gehäuses 4 angeordnet sein. Mittels der Steuer- und Bedieneinrichtung 10 ist ein Betrieb der nachfolgend erläuterten Elemente der Vorrichtung 1 steuerbar.

Die Vorrichtung 1 umfasst weiter eine erste Strahlungsquelle 11, die an der Deckenwand 9 des Deckelteils 6 angeordnet ist. Die erste Strahlungsquelle 11 ist hierbei derart angeordnet, dass die von der ersten Strahlungsquelle 11 erzeugte Strahlung in den Aufnahmebereich oder zumindest in den Teilbereich des Aufnahmebereichs 3 gestrahlt werden kann, in dem die Kontaktlinse 2 angeordnet ist. Hierbei ist eine erste Linse 12, die ein erfindungsgemäßes Mittel zur Strahlformung bildet, in Strahlrichtung vor der ersten Strahlungsquelle 11 angeordnet, um die erzeugte Strahlung auszurichten.

Weiter umfasst die Vorrichtung 1 eine zweite Strahlungsquelle 13 und eine zweite Linse 14. Die zweite Strahlungsquelle 13 ist hierbei an der Bodenwand des Bodenteils 5 angeordnet. In Strahlrichtung der von der zweiten Strahlungsquelle 13 erzeugten Strahlrichtung ist die zweite Linse 14 angeordnet, die die erzeugte Strahlung ausrichtet.

Die erste und die zweite Linse 12, 14 sind hierbei transparent für die Strahlung, die von der jeweiligen Strahlungsquelle erzeugt wird, vor der die Linse 12, 14 angeordnet ist. Insbesondere sind die Linsen 12, 14 UV-transparent.

Die erste Strahlungsquelle 11 erzeugt hierbei Strahlung mit einer Wellenlänge aus einem ersten Wellenlängenbereich von 165 nm bis 230 nm. Die erste Strahlungsquelle 11 kann insbesondere als UV-LED ausgebildet sein. Die zweite Strahlungsquelle 13 erzeugt Strahlung mit einer Wellenlänge aus einem zweiten Wellenlängenbereich von 260 nm bis 300 nm, wobei die weitere Strahlungsquelle 13 als UV-OLED ausgebildet sein kann.

Es ist vorstellbar, dass die Vorrichtung 1 eine weitere Strahlungsquelle umfasst, die Strahlung mit einer Wellenlänge aus einem dritten Wellenlängenbereich von 230 nm bis 260 nm erzeugt. Diese weitere Strahlungsquelle ist vorzugsweise als UV-Laser oder UV-LED ausgebildet. Durch die Erzeugung von Strahlung durch diese weitere Strahlungsquelle kann die Effizienz der Reinigung erhöht werden.

Die weitere Strahlungsquelle kann hierbei an der Deckenwand 9 oder an der Bodenwand angeordnet sein und Strahlung in den Aufnahmebereich 3 strahlen.

In Fig. 2 ist ein schematischer Querschnitt durch eine erfindungsgemäße Vorrichtung 1 in einer weiteren Ausführungsform dargestellt. Im Unterschied zu der in Fig. 1 dargestellten Ausführungsform ist die erste Strahlungsquelle 11 an einer ersten Seitenwand 8 und die zweite Strahlungsquelle 13 an einer der ersten Seitenwand 8 gegenüberliegenden Seitenwand 8 angeordnet. In dieser Ausführungsform sind beispielhaft keine Linsen 12, 14 vorhanden. Die Strahlungsquellen 11, 13 können hierbei insbesondere als UV-LED oder UV-OLED ausgebildet sein. Hierbei können die als UV-OLED ausgebildeten Strahlungsquellen 11, 13 sogenannte Flächenstrahler ausbilden. Ebenfalls sind die Strahlungsquellen 11, 13 derart angeordnet, dass die jeweils erzeugte Strahlung in den Aufnahmebereich 3 gestrahlt werden kann.

Es ist vorstellbar, dass die Vorrichtung 1 eine weitere Strahlungsquelle umfasst, die Strahlung mit einer Wellenlänge aus einem dritten Wellenlängenbereich von 230 nm bis 260 nm erzeugt. Diese weitere Strahlungsquelle ist vorzugsweise als UV-Laser oder UV-LED ausgebildet. Durch die Erzeugung von Strahlung durch diese weitere Strahlungsquelle kann die Effizienz der Reinigung erhöht werden.

Die weitere Strahlungsquelle kann hierbei ebenfalls an einer der Seitenwände 8 angeordnet sein und Strahlung in den Aufnahmebereich 3 strahlen.

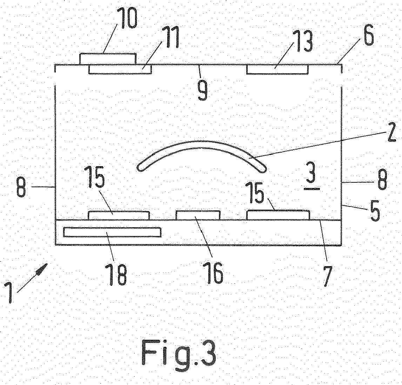

In Fig. 3 ist ein schematischer Querschnitt durch eine erfindungsgemäße Vorrichtung 1 in einer weiteren Ausführungsform dargestellt. Im Unterschied zu der in Fig. 1 dargestellten Ausführungsform sind sowohl die erste Strahlungsquelle 11 als auch die weitere Strahlungsquelle 13 an der Deckenwand 9 des Deckelteils 6 angeordnet. An der Bodenwand 7 des Bodenteils 5 sind Reflektoren 15 angeordnet, die zumindest einen Teil der von den Strahlungsquellen 11, 13 erzeugten Strahlung reflektieren. Somit kann sowohl eine Oberseite als auch eine Unterseite der Kontaktlinse 2 bestrahlt werden. Ebenfalls an der Bodenwand angeordnet ist eine Ultraschallquelle 16, die Ultraschallwellen erzeugen und in den Aufnahmebereich 3 strahlen kann.

Es ist vorstellbar, dass die Vorrichtung 1 eine weitere Strahlungsquelle umfasst, die Strahlung mit einer Wellenlänge aus einem dritten Wellenlängenbereich von 230 nm bis 260 nm erzeugt. Diese weitere Strahlungsquelle ist vorzugsweise als UV-Laser oder UV-LED ausgebildet. Durch die Erzeugung von Strahlung durch diese weitere Strahlungsquelle kann die Effizienz der Reinigung erhöht werden. Die weitere Strahlungsquelle kann hierbei ebenfalls an der Deckenwand 9 angeordnet sein und Strahlung in den Aufnahmebereich 3 strahlen.

Bei den in Fig. 1, Fig. 2 und Fig. 3 dargestellten Ausführungsformen kann jede der Strahlungsquellen 11, 13 sowie die nicht dargestellte weitere Strahlungsquelle jeweils als UV-Laser, als UV-LED oder als UV-OLED ausgebildet sein. Weiter ist die Anordnung der Strahlungsquellen 11, 13 an der Deckenwand 9, der Bodenwand oder der Seitenwand 8 exemplarisch. Insbesondere können die Strahlungsquellen 11, 13 sowie die nicht dargestellte weitere Strahlungsquelle und gegebenenfalls vorhandene Reflektoren derart im oder am Gehäuse 4 angeordnet sein, dass von der jeweilige Strahlungsquelle Strahlung in den Aufnahmebereich 3 gestrahlt werden kann.

Somit sind verschiedene Kombinationen aus Flächenstrahlern, insbesondere eine UV-OLED, und/oder Punktstrahlern, insbesondere einer UV-LED oder einer UV-Laserquelle, möglich, die Strahlung aus verschiedenen Richtungen in den Aufnahmebereich 3 emittieren können.

So können beispielsweise mindestens ein Punktstrahler von oben und mindestens ein Punktstrahler von unten in den Aufnahmebereich 3 strahlen. Auch können mindestens ein Punktstrahler von einer ersten Seite und mindestens ein Punktstrahler von einer weiteren Seite in den Aufnahmebereich 3 strahlen. Auch können mindestens ein Punktstrahler von oben oder unten und mindestens ein Punktstrahler von einer der beiden Seiten in den Aufnahmebereich 3 strahlen.

Auch können beispielsweise mindestens ein Flächenstrahler von oben und mindestens ein Flächenstrahler von unten in den Aufnahmebereich 3 strahlen. Auch können mindestens ein Flächenstrahler von einer ersten Seite und mindestens ein Flächenstrahler von einer weiteren Seite in den Aufnahmebereich 3 strahlen. Auch können mindestens ein Flächenstrahler von oben oder unten und mindestens ein Flächenstrahler von einer der beiden Seiten in den Aufnahmebereich 3 strahlen.

Weiter können beispielsweise mindestens ein Flächenstrahler von oben oder unten und mindestens ein Punktstrahler von unten oder oben in den Aufnahmebereich 3 strahlen. Auch können mindestens ein Flächenstrahler von der ersten Seite oder der weiteren Seite und mindestens ein Punktstrahler von der weiteren Seite oder der ersten Seite in den Aufnahmebereich 3 strahlen. Auch können mindestens ein Flächenstrahler von oben oder unten und mindestens ein Punktstrahler von einer der beiden Seiten in den Aufnahmebereich 3 strahlen. Auch können mindestens ein Punktstrahler von oben oder unten und mindestens ein Flächenstrahler von einer der beiden Seiten in den Aufnahmebereich 3 strahlen.

Weiter können bei den in den Fig. 1, Fig. 2 und Fig. 3 dargestellten Ausführungsformen die Wände, insbesondere die Deckenwand 9, die Bodenwand und/oder die Seitenwände 8, reflektierend, insbesondere verspiegelt, ausgebildet sein.

Bei den in Fig. 1, Fig. 2 und Fig. 3 dargestellten Ausführungsformen ist jeweils nur eine Strahlungsquelle 11, 13 dargestellt. Es ist selbstverständlich möglich, dass die Vorrichtung 1 mehr als die zwei dargestellten Strahlungsquellen 11, 13 umfasst. Insbesondere kann die Vorrichtung 1 eine erste Menge von mindestens zwei Strahlungsquellen umfassen, wobei durch jede Strahlungsquelle der ersten Menge Strahlung mit einer Wellenlänge aus dem ersten Wellenlängenbereich emittierbar ist. Diese Strahlungsquellen können an verschiedenen Positionen angeordnet sein. Alternativ kann die Vorrichtung 1 eine zweite Menge von mindestens zwei Strahlungsquellen umfassen, wobei durch jede Strahlungsquelle der zweiten Menge Strahlung mit einer Wellenlänge aus dem zweiten Wellenlängenbereich emittierbar ist. Diese Strahlungsquellen können an verschiedenen Positionen angeordnet sein. Generell, insbesondere aber auch in diesem Fall, ist es möglich, die Vorrichtung 1 in mindestens zwei voneinander verschiedenen Betriebsmodi zu betreiben, wobei sich verschiedene Betriebsmodi in dem Aktivierungszustand mindestens einer Strahlungsquelle 11, 13 und/oder in der Intensität der von einer ausgewählten Strahlungsquelle 11, 13 im jeweiligen Betriebsmodus emittierten Strahlung unterscheiden.

### Bezugszeichenliste

- 1: Vorrichtung
- 2: Kontaktlinse
- 3: Aufnahmebereich
- 4: Gehäuse
- 5: Bodenteil
- 6: Deckelteil
- 7: Bodenwand
- 8: Seitenwand
- 9: Deckenwand
- 10: Steuer- und Bedieneinrichtung
- 11: erste Strahlungsquelle
- 12: erste Linse
- 13: zweite Strahlungsquelle
- 14: zweite Linse
- 15: Reflektor
- 16: Ultraschallquelle
- 18: Energiespeichereinrichtung

## Patentansprüche

1. Vorrichtung zur Reinigung eines Medizinprodukts, wobei die Vorrichtung (1) mindestens einen Aufnahmebereich (3) für das Medizinprodukt aufweist, wobei die Vorrichtung (1) eine erste Strahlungsquelle (11) umfasst, wobei durch die erste Strahlungsquelle (11) Strahlung mit einer Wellenlänge aus einem ersten Wellenlängenbereich von 165 nm bis 230 nm emittierbar ist, wobei zumindest ein Teil der Strahlung der ersten Strahlungsquelle (11) in zumindest einen Teilbereich des Aufnahmebereichs (3) strahlbar ist, wobei die Vorrichtung (1) mindestens eine zweite Strahlungsquelle (13) umfasst, **dadurch gekennzeichnet, dass** durch die zweite Strahlungsquelle (13) Strahlung mit einer Wellenlänge aus einem zweiten Wellenlängenbereich von 260 nm bis 300 nm emittierbar ist, wobei zumindest ein Teil der Strahlung der zweiten Strahlungsquelle (13) in zumindest einen Teilbereich des Aufnahmebereichs (3) strahlbar ist.

2. Vorrichtung nach Anspruch 1, **dadurch gekennzeichnet, dass** die Vorrichtung (1) mindestens eine weitere Strahlungsquelle umfasst, wobei durch die mindestens eine weitere Strahlungsquelle Strahlung mit einer Wellenlänge aus einem dritten Wellenlängenbereich von 230 nm bis 260 nm emittierbar ist, wobei zumindest ein Teil der Strahlung der mindestens einen weiteren Strahlungsquelle in zumindest einen Teilbereich des Aufnahmebereichs (3) strahlbar ist.

3. Vorrichtung nach Anspruch 1 oder 2, **dadurch gekennzeichnet, dass** die erste Strahlungsquelle (11) als UV-LED, als UV-OLED oder als UV-Laserquelle ausgebildet ist und/oder dass die zweite Strahlungsquelle (13) als UV-LED, als UV-OLED oder als UV-Laserquelle ausgebildet ist.

4. Vorrichtung nach einem der vorangegangenen Ansprüche, **dadurch gekennzeichnet, dass** eine Summe der von aktivierten oder aktivierbaren Strahlungsquellen (11, 13) emittierbaren Strahlung größer als oder gleich 2,0 µJ/mm² ist.

5. Vorrichtung nach einem der vorangegangenen Ansprüche, **dadurch gekennzeichnet, dass** die Vorrichtung (1) zusätzlich eine Ultraschallquelle (16) umfasst, wobei zumindest ein Teil des Ultraschallsignals in zumindest einen Teilbereich des Aufnahmebereichs (3) strahlbar ist.

6. Vorrichtung nach einem der vorangegangenen Ansprüche, **dadurch gekennzeichnet, dass** die Vorrichtung (1) mindestens ein Mittel zur Strahlführung und/oder -formung umfasst.

7. Vorrichtung nach einem der vorangegangenen Ansprüche, **dadurch gekennzeichnet, dass** der Aufnahmebereich (3) zumindest einseitig durch ein Wandelement begrenzt ist, wobei die mindestens eine Strahlungsquelle (11, 13) im oder am Wandelement angeordnet ist.

8. Vorrichtung nach einem der vorangegangenen Ansprüche, **dadurch gekennzeichnet, dass** das eine Bestrahlungsdauer und/oder Bestrahlungsleistung einstellbar ist.

9. Vorrichtung nach einem der vorangegangenen Ansprüche, **dadurch gekennzeichnet, dass** die Vorrichtung (1) einen Aufnahmebehälter umfasst, wobei der Aufnahmebehälter den Aufnahmebereich (3) aufweist, wobei die erste und die zweite Strahlungsquelle (11, 13) im oder am Aufnahmebehälter angeordnet sind.

10. Vorrichtung nach einem der vorangegangenen Ansprüche, **dadurch gekennzeichnet, dass** die erste und/oder die zweite und/oder die mindestens eine weitere Strahlungsquelle (11, 13) als Flächenstrahler ausgebildet ist.

11. Vorrichtung nach einem der vorangegangenen Ansprüche, **dadurch gekennzeichnet, dass** die Vorrichtung (1) eine erste Menge von mindestens zwei Strahlungsquellen umfasst, wobei durch jede Strahlungsquelle der ersten Menge Strahlung mit einer Wellenlänge aus dem ersten Wellenlängenbereich emittierbar ist, und/oder eine zweite Menge von mindestens zwei Strahlungsquellen umfasst, wobei durch jede Strahlungsquelle der zweiten Menge Strahlung mit einer Wellenlänge aus dem zweiten Wellenlängenbereich emittierbar ist.

12. Vorrichtung nach einem der vorangegangenen Ansprüche, **dadurch gekennzeichnet, dass** die Vorrichtung (1) in mindestens zwei voneinander verschiedenen Betriebsmodi betreibbar ist, wobei sich verschiedene Betriebsmodi in dem Aktivierungszustand mindestens einer Strahlungsquelle und/oder in der Intensität der von einer ausgewählten Strahlungsquelle im jeweiligen Betriebsmodus emittierten Strahlung unterscheiden.

13. Verfahren zur Reinigung eines Medizinprodukts, wobei der Medizinprodukt in einem Aufnahmebereich (3) angeordnet wird, wobei Strahlung mit einer Wellenlänge aus einem ersten Wellenlängenbereich von 165 nm bis 230 nm in zumindest einen Teilbereich des Aufnahmebereichs (3) eingestrahlt wird, **dadurch gekennzeichnet, dass** Strahlung mit einer Wellenlänge aus einem weiteren Wellenlängenbereich von 260 nm bis 300 nm in zumindest einen Teilbereich des Aufnahmebereichs (3) eingestrahlt wird.

14. Verfahren nach Anspruch 13, **dadurch gekennzeichnet, dass** zusätzlich eine Reinigungslösung im Aufnahmebereich (2) angeordnet wird.

15. Verfahren zur Reinigung eines Gehäuses (4) einer Vorrichtung zur Reinigung eines Medizinprodukts gemäß einem der Ansprüche 1 bis 12 und/oder zur Reinigung von einer Reinigungslösung, wobei Strahlung mit einer Wellenlänge aus einem ersten Wellenlängenbereich von 165 nm bis 230 nm in zumindest einen Teilbereich des Aufnahmebereichs (3) eingestrahlt wird und/oder Strahlung mit einer Wellenlänge aus einem zweiten Wellenlängenbereich von 260 nm bis 300 nm in zumindest einen Teilbereich des Aufnahmebereichs (3) eingestrahlt wird, wenn kein Gegenstand im Aufnahmebereich (3) angeordnet ist.
